(19) Europäisches Patentamt
European Patent Office
Office européen des brevets

(11) Publication number: **0 234 431**
**A2**

(12)

# EUROPEAN PATENT APPLICATION

(21) Application number: **87101992.3**

(22) Date of filing: **12.02.87**

(51) Int. Cl.4: **C07D 311/92**, A61K 31/35

The applicant has filed a statement in accordance with Rule 28 (4) EPC (issue of a sample only to an expert). Accession number(s) of the deposit(s): FERM BP-1218.

(30) Priority: **14.02.86 JP 28935/86**

(43) Date of publication of application: **02.09.87 Bulletin 87/36**

(84) Designated Contracting States: **AT BE CH DE ES FR GB GR IT LI LU NL SE**

(71) Applicant: **HOECHST JAPAN LIMITED**
**10-16, 8-chome, Akasaka, Minato-ku**
**Tokyo(JP)**

(72) Inventor: **Kurobane, Itsuo**
**2-23-206, 1, Sayamadai 2-chome**
**Sayama-shi Saitama-ken(JP)**
Inventor: **Senda, Shunji**
**Nihon-Hoechst-Ryo 7-22, Minamidai 3-chome**
**Kawagoe-shi Saitama-ken(JP)**
Inventor: **Matsuo, Akihiko**
**8-403, 1, Namiki 7-chome**
**Tokorozawa-shi Saitama-ken(JP)**
Inventor: **Fukuda, Akio**
**12-16, Nakameguro 3-chome**
**Meguro-ku Tokyo(JP)**

(74) Representative: **Becker, Heinrich Karl Engelbert, Dr. et al**
**HOECHST AKTIENGESELLSCHAFT Central Patent Department P.O. Box 80 03 20**
**D-6230 Frankfurt am Main 80(DE)**

(54) **5-Hydroxydihydrofusarubin, a process for its preparation and its use as a medicament.**

(57) The present invention relates to 5-Hydroxydihydrofusarubin which possesses antimicrobial and antitumor activities. It is prepared by aerobically cultivating a 5-hydroxydihydrofusarubin-producing microorganism belonging to the genus Fusarium, e.g. Fusarium solani.

EP 0 234 431 A2

**5-Hydroxydihydrofusarubin, a process for its preparation and its use as a medicament.**

The present invention relates to 5-hydroxydihydrofusarubin and a process for preparing the same.

5-Hydroxydihydrofusarubin is a novel compound which is useful as an antimicrobial or antitumor agent, or as a starting material for such agents.

It has heretobefore been reported that fusarubin (G. P. Arsenault, Canad. J. Chem. 43, 2423, 1965), dihydrofusarubin (Kurobane et al., Canad. J. Chem. 56, 1593, 1978) and ethyl ethers of them (Kurobane et al., J. Antibiotics, 33, 1376, 1980) were isolated from culture broths of Fursarium solani belonging to Fungi Imperfecti and that these possess antimicrobial activities. In addition, there have been isolated from the above-mentioned culture broths javanicin (H. R. V. Arnstein et al., J. Chem. Soc. 1021, 1947), norjavanicin - (W. S. Chilton, J. Org. Chem. 33, 4299, 1968) and bostrycoidin (G. P. Arsenault, Tetrahedron Lett., 4033, 1965).

These metabolites, however, are not strong enough in antimicrobial activity, and not high enough in solubility in water so that discovery of metabolites with better pharmacological activities has been desired.

As a result of extensive studies to isolate metabolites with better pharmacological activities from culture broths of microorganisms of the genus Fusarium, 5-hydroxydihydrofusarubin has been isolated from the above-mentioned culture. The present invention has been completed on the basis of the above discovery.

The compound of the invention 5-hydroxydihydrofusarubin is represented by the formula (I)

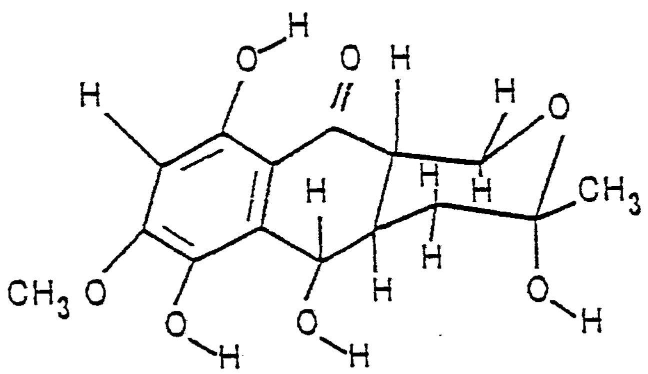

(I)

The invention relates further to a process for preparing 5-hydroxydihydrofusarubin by aerobically cultivating a 5-hydroxydihydrofusarubin-producing microorganism belonging to the genus Fusarium and isolating 5-hydroxydihydrofusarubin from the culture broth.

The present invention relates particularly to cultivating Fusarium solani f. sp. pisi T-127 (called T-127 hereinbelow) under aerobical conditions and isolating 5-hydroxydihydrofusarubin from the culture thus obtained.

The above-mentioned strain was received from Professor Hans D. VanEtten of Department of Phytopathology, Faculty of Agriculture, Cornell University, U.S.A. Mycological properties and taxonomical description of the strain are reported in Phytopathology, 68, 1552-1556, 1978. T-127 was deposited on October 5, 1985 with Fermentation Research Institute, Agency of Industrial Science and Technology, Ministry of International Trade and Industry under the domestic deposit number 8478, international deposit No. FERM BP-1218.

Results of an assimilation test on T-127 for various carbon and nitrogen sources and yields of 5-hydroxydihydrofusarubin (I) then produced are as follows:

Table 1 Assimilation of carbon sources

| Carbon source | Assimilation | 5-Hydroxydihydro-fusarubin (I) |
|---|---|---|
| D-Glucose | +++ | +++ |
| D-Fructose | +++ | ++ |
| D-Xylose | ++ | ++ |
| D-Mannitol | +++ | + |
| D-Raffinose | +++ | + |
| D-Arabinose | +++ | + |
| L-Ramnose | +++ | + |
| Lactose | +++ | + |
| i-Inositol | +++ | + |
| Sucrose | +++ | +++ |
| Maltose | +++ | +++ |

(Notes) +: Slight growth or production

++: Moderate growth or production

+++: Vigorous growth or production

A carbon source was used in a proportion of 30 g/l, and as a nitrogen source casein was used in a proportion of 4.6 g/l. Inorganic salts were used in the same proportions as in Examples, and pH was adjusted to 6.0 before autoclave. The cultivation was carried out at 27°C for 3 days. Mycelia were separated by centrifugation, and 5-hydroxydihydrofusarubin (I) was extracted with ethyl acetate. A high production of 5-hydroxydihydrofusarubin (I) was observed with D-glucose, sucrose or maltose.

Table 2 Assimilation of nitrogen sources

| Nitrogen source | Assimilation | 5-Hydroxydihydrofusarubin (I) |
|---|---|---|
| Ammonium chloride | + | + |
| Ammonium acetate | +++ | +++ |
| Ammonium tartrate | +++ | +++ |
| Ammonium nitrate | ++ | + |
| Sodium nitrate | +++ | + |
| Urea | +++ | + |
| Yeast extract | +++ | ++ |
| Soybean meal | +++ | ++ |
| Casein | +++ | +++ |
| Polypepton | +++ | ++ |
| Skim milk | ++ | ++ |

The inorganic nitrogen sources and urea were used in a proportion of 0.025 mol/l, respectively, and other organic nitrogen sources were used in a proportion of 4.6 g/l, respectively. As a carbon source maltose was used in a proportion of 30 g/l. Other conditions were exactly the same as in the experiment for assimilation of carbon sources.

The yeast extract used was yeast extract for medium manufactured by Oriental Yeast Co. Ltd., the soybean meal was defatted soybean powder manufactured by Fuji Purina Protein Ltd., casein was one manufactured by Nakarai Chemicals Ltd., polypepton was one manufactured by Daigo Eiyo Kagaku Ltd. and skim milk was defatted milk powder manufactured by Yukijirushi Nyugyo Ltd.

A high production of 5-hydroxydihydrofusarubin was observed markedly with ammonium acetate, ammonium tartrate or casein.

Physicochemical properties of 5-hydroxydihydrofusarubin (I) produced by the present invention are as follows:

(1) Appearance: Orange plates

(2) Molecular formula: $C_{15}H_{18}O_7$

(3) Molecular weight: 310

(4) Melting point: 192-193°C

(5) Optical rotation: -171.4 (in 0.06% acetone solution)

(6) Ultraviolet and visible absorption spectrum: in 95% ethanol solution: $\lambda_{max}$ (nm) (log$\epsilon$) 211(4.17), 245(4.08), 284(3.97), 359(3.88)

(7) Infrared absorption spectrum:

The infrared absorption spectrum measured in potassium bromide tablet is shown in Fig. 1.

(8) $^1$H-NMR:

$^1$H-NMR measured in deuteriumchloroform is shown in Fig. 2. Chemical shifts were assigned as shown below.

| Position of proton | Coupling | Chemical shift (ppm) |
|---|---|---|
| $H-1_{ax}$ | dd | 3.81 |
| $H-1_{eq}$ | dd | 4.01 |
| $H-4_{ax}$ | dd | 1.97 |
| $H-4_{eq}$ | dd | 2.02 |
| H-4a | dddd | 2.94 |
| H-5 | dd | 5.34 |
| H-8 | s | 6.49 |
| H-10a | dddd | 3.01 |
| $3-CH_3$ | s | 1.60 |
| $7-OCH_3$ | s | 3.96 |
| 6-OH | s | 5.59 |
| 9-OH | s | 12.00 |

(9) $^{13}C$-NMR:

$^{13}C$-NMR measured in deuteriumchloroform is shown in Fig. 3. Chemical shifts were assigned as shown below.

| Position of carbon | Coupling. | Chemical shift (ppm) |
|---|---|---|
| C-1 | dt | 62.77 |
| C-3 | m | 106.25 |
| C-4 | t | 40.15 |
| C-4a | d | 38.96 |
| C-5 | dd | 72.00 |
| C-5a | bs | 121.44 |
| C-6 | dd | 137.86 |
| C-7 | m | 154.68 |
| C-8 | dd | 100.18 |
| C-9 | dd | 158.69 |
| C-9a | m | 107.25 |
| C-10 | dd | 203.10 |
| C-10a | d | 45.08 |
| C-11 | q | 23.87 |
| C-12 | q | 56.37 |

(10) Solubility:

Soluble in chloroform, methylene chloride, ethyl acetate, acetone, ethanol or methanol. Sparingly soluble in hexane or petroleum ether.

Solubility in water: Insoluble under acidic or neutral condition, and soluble under alkaline condition.

5-Hydroxydihydrofusarubin with the above-mentioned formula (I) is obtained by aerobically cultivating a 5-hydroxydihydrofusarubin-producing microorganism belonging to the genus Fusarium.

One of examples as the above-mentioned 5-hydroxydihydrofusarubin (I)-producing microorganism is Fusariumsolani (synonymous with Fusarium martii ), of which T-127 as described above is preferably employed. Any of the strains belonging to the genus Fusarium other than the above which produce 5-hydroxydihydrofusarubin may be employed. Also may be employed artificial and naturally-occuring mutants of these strains, the former being produced by ultraviolet irradiation or by treatment with a mutagenic compound used for mutation of microorganisms such as nitrosoguanidin.

Cultivation in the invention is carried out under aerobic conditions using a conventional aerobic cultivation method such as, reciprocal or rotational shaking cultivation, agitating cultivation under aeration or stationary cultivation. If defoaming is needed during sterization of the medium or during cultivation, a defoaming agent such as silicon oil or a surface-active agent can be added to the medium usually at a level in the range of 0.1-0.01%.

Any of those media which are suitable for the growth of fungi and are capable of producing 5-hydroxy dihydrofusarubin (I) as described above may be employed without particular restriction. As a carbon source are preferably employed saccharides such as, glucose, fructose, xylose, sucrose and maltose. As a nitrogen source are employed, for example, casein, yeast extract, soybean meal, polypepton and skim milk, as well as urea, ammonium salts and nitrates. In addition, minor nutrients such as inorganic salts, for example, of magnesium, calcium, sodium, potassium, iron, manganese and the like, and vitamins, for example, vitamin $B_1$, calcium pantothenate and the like can be supplemented.

Culture conditions such as pH of the medium and cultivation temperature may be variable appropriately in such a range as producing 5-hydroxydihydrofusarubin (I). In the case of liquid shaking or aerating agitation cultivation, a cultivation at a pH of 3-8 (optimum pH 5.5-6.5) and a cultivation temperature of 20-30°C (optimum temperature 23-25°C) for a cultivation period of 2-10 days (optimum period of time 3-5 days) is suitable. 5-Hydroxydihydrofusarubin (I) is accumulated in the culture broth thus obtained.

After completion of the cultivation, desired 5-hydroxydihydrofusarubin (I) is isolated from the culture broth by a conventional method. For example, the mycelium is removed from the culture broth by centrifugation, the filtrate is extracted with an appropriate solvent such as ethyl acetate or methylene chloride, and purification is performed by absorption chromatography, for example, silica gel chromatography.

Results of an antimicrobial test and an antitumor test for 5-hydroxydihydrofusarubin (I) of the invention are shown below.

Antimicrobial test:

Minimum inhibitory concentrations (MIC) of 5-hydroxydihydrofusarubin (I) against bacteria and fungi were determined by the agar plate-dilution method. The results are shown in Table 3.

Table 3 Minimum inhibitory concentration

| Test organism | | MIC(μg/ml) |
|---|---|---|
| *Staphylococcus aureus* | FDA209PJC1 | 100 |
| *Bacillus subtilis* | ATCC 6633 | 100 |
| *Streptococcus pneumoniae* | DP-1 | 50 |
| *Candida albicans* | C-a-30 | 100 |
| *Saccharomyces cereviciae* | ATCC 9763 | 50 |

As demonstrated above in Table 3, 5-hydroxydihydrofusarubin possesses antimicrobial activities against Gram-positive bacteria and fungi.

Antitumor test:

An antitumor test was carried out for 5-hydroxy dihydrofusarubin. Half value ($IC_{50}$) inhibiting growth of mouse leukemia L1210 cells in suspension ($1 \times 10^6$/mouse) was as follows:

| | $IC_{50}$ (μg/ml) |
|---|---|
| 5-Hydroxydihydrofusarubin | $9.4 \pm 0.9$ |

It is evident from the results of the antimicrobial test and the antitumor test described above that 5-hydroxydihydrofusarubin exerts antimicrobial and antitumor activities. As an antimicrobial agent the 5-hydroxydihydrofusarubin can be used in the absence or presence of a carrier in pharmaceuticals, agrochemicals, food additives or the like. Its antitumor activity allows us to use the above-mentioned compound in the absence or presence of a carrier, or in the form of a complex bound to antibody or polymer as an anticancer agent.

An example is given below to describe the invention in more details.

Example

In a 500-ml Erlenmeyer flask was placed 100 ml of a medium with a composition shown in Table 4, and the flask was capped with cotton and sterilized in an autoclave at 121 °C for 20 min.

Table 4 Composition of the medium

| Component | Concentration (g/l) |
|---|---|
| Maltose | 40 |
| Ammonium tartrate | 4.6 |
| $KH_2PO_4$ | 1.0 |
| $MgSO_4 \cdot 7H_2O$ | 0.5 |
| NaCl | 0.1 |
| $CaCl_2$ | 0.1 |
| $FeSO_4 \cdot 7H_2O$ | 0.01 |
| $ZnSO_4 \cdot 7H_2O$ | 0.0088 |
| $CuSO_4 \cdot 5H_2O$ | 0.0004 |
| $MnSO_4$ | 0.00006 |
| $H_3BO_3$ | 0.00006 |
| $(NH_4)_6Mo_7O_{24} \cdot 4H_2O$ | 0.00004 |

The above-mentioned medium was inoculated with Fusarium solani f. sp. pisi T-127 (Institute of Industrial Technology of Microbiology Deposit No. 8478), and the inoculum was cultivated by rotationary shaking at 220 rpm at 27°C for 3 days. The seed culture was transplanted onto the medium described in Table 4 and sterilized beforehand, and the second seed cultivation was initiated. The cultivation was carried out for one day under the same conditions as described above. The seed culture was transplanted onto 40 lit. of a medium planced in a 80-lit. jar fermenter. The medium was the same as described in Table 4 except that the ammonium tartrate was replaced by casein. The cultivation was carried out with aeration of 20 lit. per minute and rotation at 150 per minute at 27°C with addition as needed of a defoaming agent (® Desmophen Type 3600 manufactured by Hoechst AG, Germany). After 2 days of cultivation, the culture was stopped, and the mycelium was separated by centrifugation.

The pH of the filtrate was adjusted to 6.5 with 1N NaOH solution, followed by extraction with an equivalent amount of ethyl acetate. The extract was washed with distilled water and concentrated by an evaporator. Crude extract concentrated to dryness was washed with petroleum ether and then dissolved in methylene chloride. Soluble fraction was again concentrated by an evaporator. The crude fraction thus concentrated was subjected to silica gel column (Kiesel gel 60 manufactured by Merck) and developed with a mixed solvent of methylene chloride-ethyl acetate. 5-Hydroxydihydrofusarubin was eluted with the mixed solvent of a mixing ratio of 9 : 1. The eluted fraction was further subjected to high performance liquid chromatography (Silica gel S manufactured by Kusano Kagaku) and developed with the mixed solvent mentioned above. The fractions eluted with 9 : 1 mixed solvent were concentrated to dryness, followed by crystallization in a methylene chloride-ethanol (2 : 1) mixed solvent. From 40 lit. of the culture broth was obtained 40.3 mg of 5-hydroxydihydrofusarubin (I).

**Claims**

(1) 5-Hydroxydihydrofusarubin represented by the formula (I)

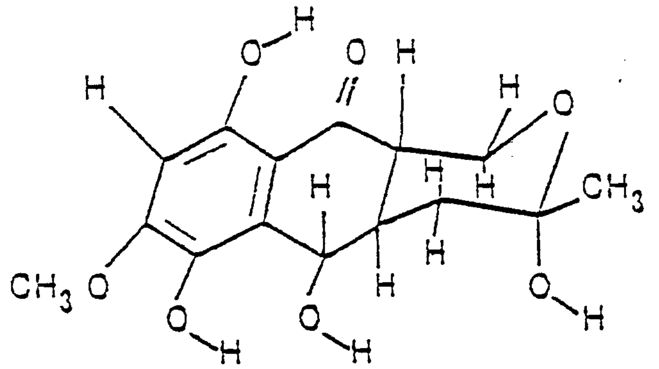

(I)

(2) A process for preparing 5-hydroxydihydrofusarubin represented by the formula (I)

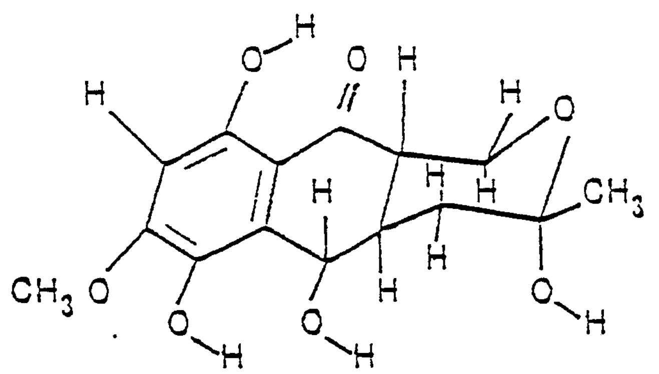

(I)

which comprises aerobically cultivating a 5-hydroxydihydrofusarubin-producing microorganism belonging to the genus Fusarium in a nutrient medium containing carbon sources, nitrogen sources and optionally inorganic salts and vitamins and isolating 5-hydroxydihydrofusarubin from the culture broth, by conventional methods.

(3) A process as defined in claim 2 wherein the microorganism is cultivated at a pH of 3-8, a temperature of 20-30° C and over a period of 2-10 days, and the desired compound is isolated from the culture broth by extraction with an appropriate solvent and subsequent application of a chromatographical method to the extract.

(4) A process as defined in claim 3 wherein the microorganism of the genus Fusarium is Fusarium solani (FERM BP-1218).

(5) A pharmaceutical composition comprising as active ingredient an effective amount of a compound as defined in claim 1 in association with a pharmaceutically acceptable carrier.

(6) Use of a compound as defined in claim 1 for the preparation of a medicament having antimicrobial and/or anti-cancer activities.

Claims for the following contracting states: Austria, Greece and Spain

(1) A process for preparing 5-hydroxydihydrofusarubin represented by the formula (I)

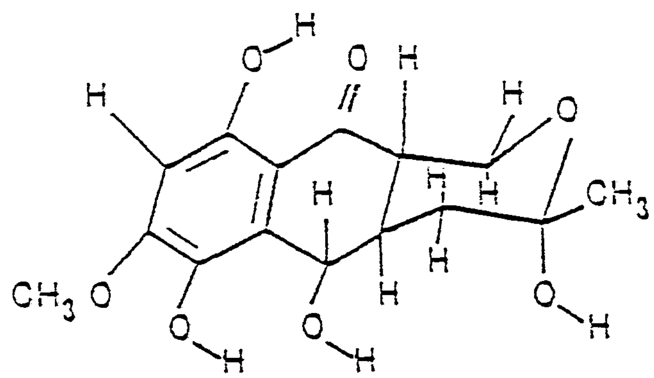

(I)

which comprises aerobically cultivating a 5-hydroxydihydrofusarubin-producing microorganism belonging to the genus Fusarium in a nutrient medium containing carbon sources nitrogen sources and optionally inorganic salts and vitamins and isolating 5-hydroxydihydrofusarubin from the culture broth, by conventional methods.

(2) A process as defined in claim 2 wherein the microorganism is cultivated at a pH of 3-8, a temperature of 20-30° C and over a period of 2-10 days, and the desired compound is isolated from the culture broth by extraction with an appropriate solvent and subsequent application of a chromatographical method to the extract.

(3) A process as defined in claim 2 wherein the microorganism of the genus Fusarium is Fusarium solani (FERM BP-1218).

(4) Use of a compound as defined in claim 1 for the preparation of a medicament having antimicrobial and/or anti-cancer activities.

FIG. 1

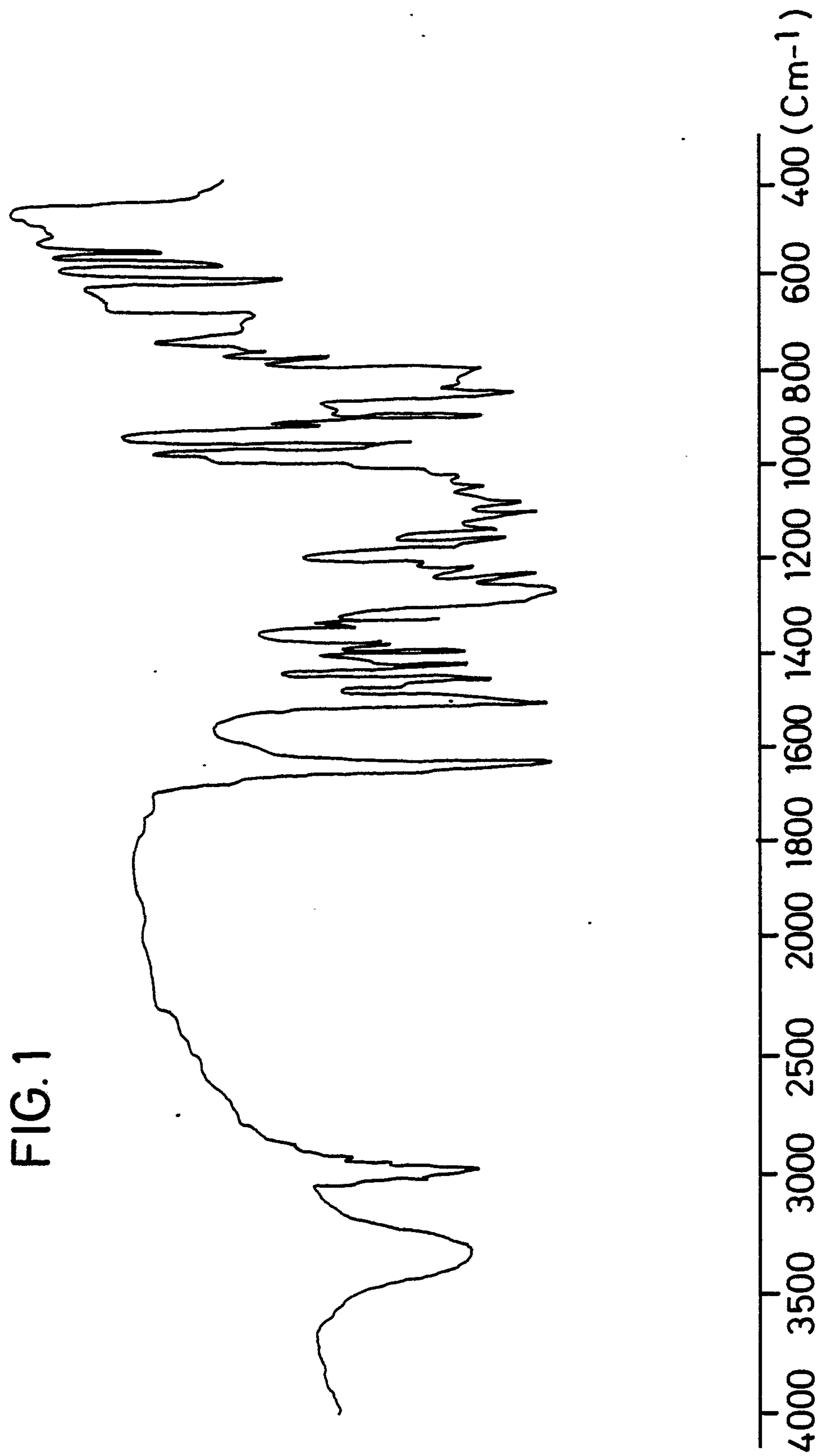
400
600
800
1000
1200
1400
1600
1800
2000
2500
3000
3500
4000
(Cm-1)

FIG. 2

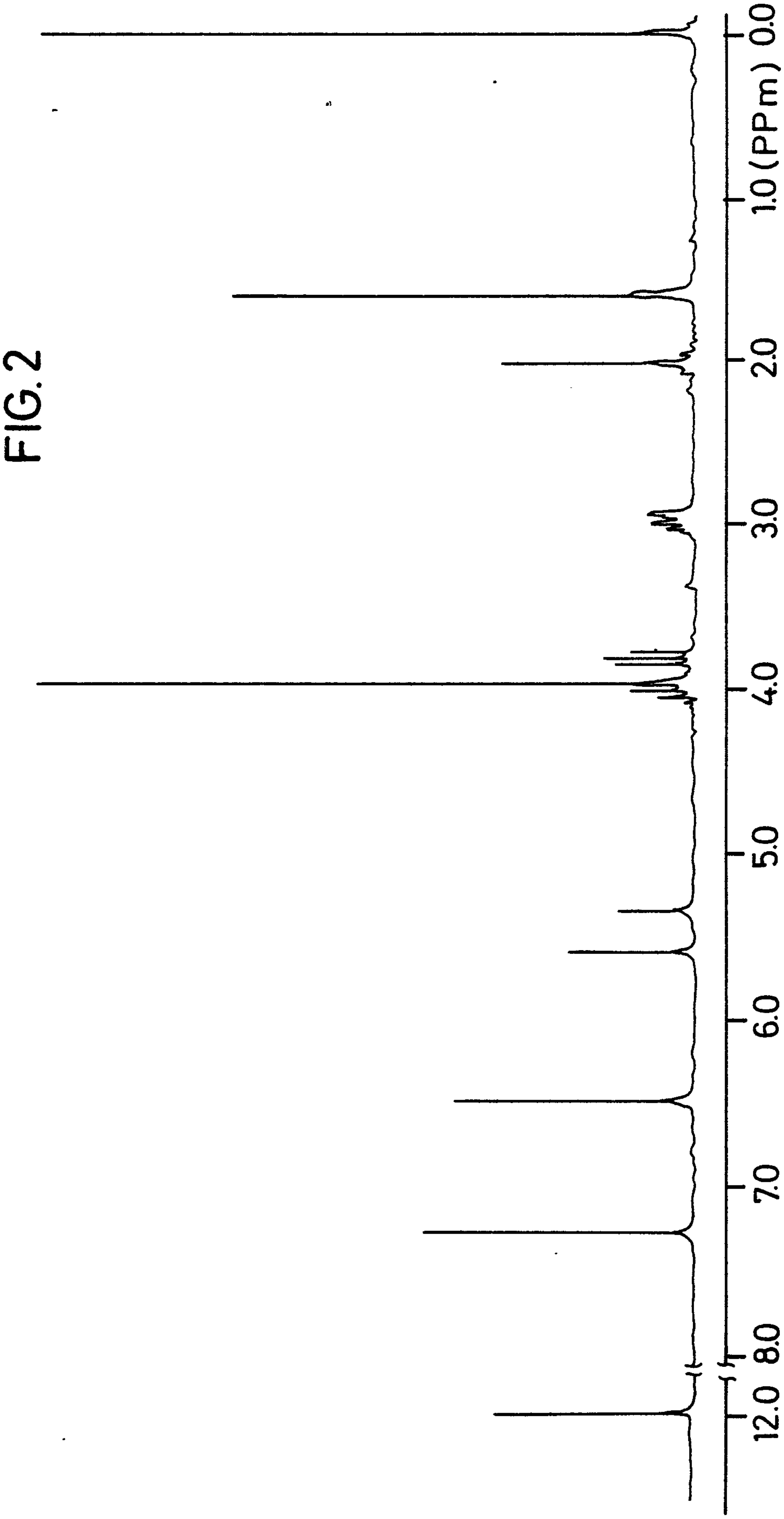

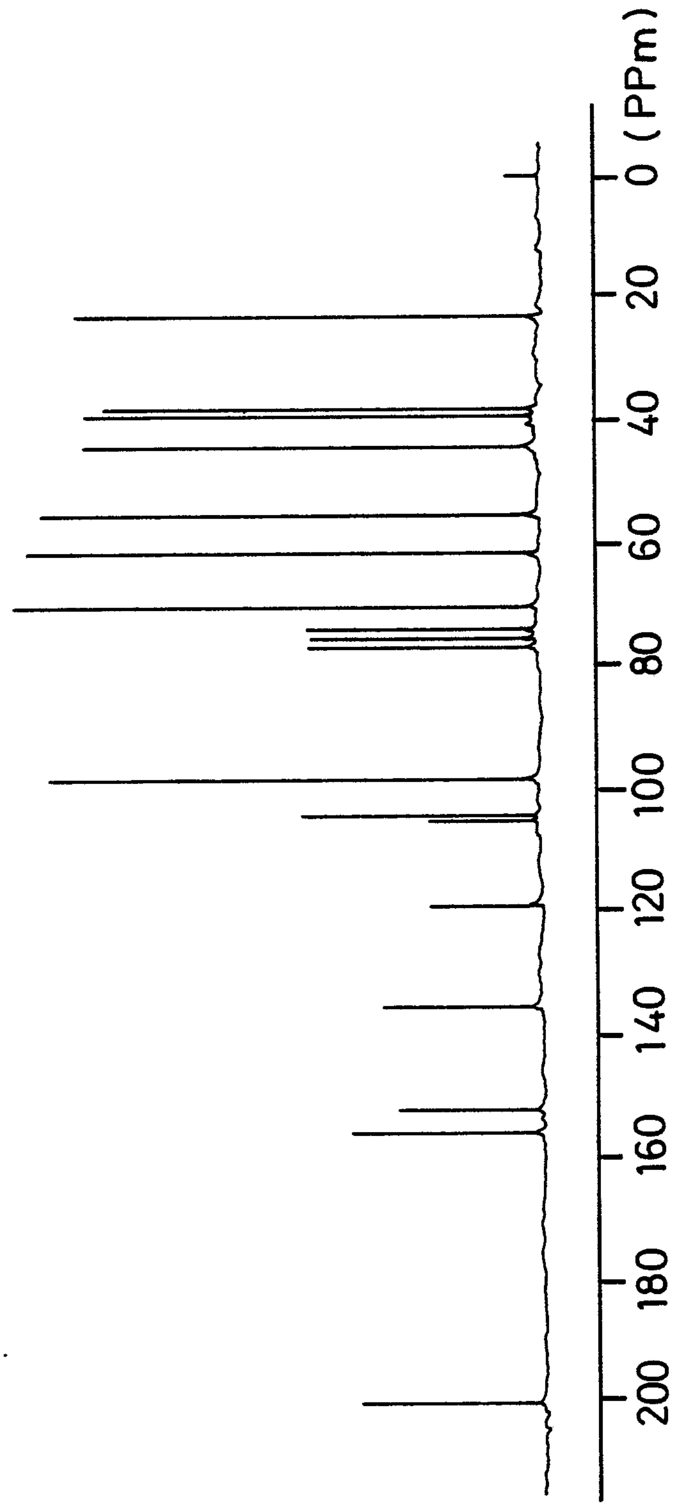


FIG. 3